(19) Europäisches Patentamt
European Patent Office
Office européen des brevets

(11) EP 2 688 861 B1

(12) **EUROPÄISCHE PATENTSCHRIFT**

(45) Veröffentlichungstag und Bekanntmachung des Hinweises auf die Patenterteilung:
**17.08.2016 Patentblatt 2016/33**

(21) Anmeldenummer: **12710259.8**

(22) Anmeldetag: **21.03.2012**

(51) Int Cl.:
***C07C 209/48*** *(2006.01)*

(86) Internationale Anmeldenummer:
**PCT/EP2012/055024**

(87) Internationale Veröffentlichungsnummer:
**WO 2012/126956 (27.09.2012 Gazette 2012/39)**

(54) **VERFAHREN ZUR HYDRIERUNG VON NITRILEN**

METHOD FOR HYDROGENATING NITRILES

PROCÉDÉ D’HYDROGÉNATION DE NITRILES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.03.2011 EP 11159147**

(43) Veröffentlichungstag der Anmeldung:
**29.01.2014 Patentblatt 2014/05**

(73) Patentinhaber: **BASF SE**
**67056 Ludwigshafen am Rhein (DE)**

(72) Erfinder:
- **WIGBERS, Christof, Wilhelm**
  **68167 Mannheim (DE)**
- **MÜLLER, Christoph**
  **68165 Mannheim (DE)**
- **MÄGERLEIN, Wolfgang**
  **68165 Mannheim (DE)**
- **KUBANEK, Petr**
  **68163 Mannheim (DE)**
- **HEIDEMANN, Thomas**
  **68519 Viernheim (DE)**
- **MELDER, Johann-Peter**
  **67459 Böhl-Iggelheim (DE)**
- **KRUG, Thomas**
  **67550 Worms (DE)**
- **BEY, Oliver**
  **67150 Niederkirchen (DE)**

(74) Vertreter: **BASF IP Association**
**BASF SE**
**ZRX-C6**
**67056 Ludwigshafen (DE)**

(56) Entgegenhaltungen:
**EP-A1- 0 449 089** **WO-A1-03/035250**
**WO-A1-2007/128803**

- **CHARLES N SATTETFIELD: "TRICKLE-BED REACTORS", AICHE JOURNAL, JOHN WILEY & SONS, INC, US, vol. 21, no. 2, 1 March 1975 (1975-03-01), pages 209-228, XP003035933, ISSN: 0001-1541**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 688 861 B1

Printed by Jouve, 75001 PARIS (FR)

## Beschreibung

Die vorliegende Erfindung betrifft ein kontinuierliches Verfahren zur Hydrierung von Nitrilen mit Wasserstoff in Gegenwart eines Katalysators in einem Reaktor, wobei der Katalysator in einem Festbett angeordnet ist, dadurch gekennzeichnet, dass die Querschnittsbelastung im Reaktor im Bereich von 5 kg/($m^2$ s) bis 50 kg/($m^2$ s) liegt und als Nitrile 3-(Dimethylamino)propionitril oder Isophoronnitrilimin und/oder Isophoronnitril eingesetzt werden.

Ein weiterer Gegenstand der vorliegenden Erfindung ist ein Verfahren zur Herstellung von Folgeprodukten von Isophorondiamin (IPDA) bzw. N,N-Dimethylaminopropylamin (DMAPA) aus erfindungsgemäß hergestellten Aminen.

Bei der Hydrierung von Nitrilen zu den entsprechenden Aminen ist es häufig erforderlich einen hohen Umsetzungsgrad bezüglich der eingesetzten Nitrile zu erzielen, da nicht umgesetzte oder nur teilweise umgesetzte Nitrile nur schwer abzutrennen sind, Nebenreaktionen eingehen können und in den Folgeanwendungen zu unerwünschten Eigenschaften, wie Geruch und Verfärbung, führen können. Es ist weiterhin häufig wünschenswert eine hohe Selektivität bezüglich der Bildung von primären Aminen aus primären Nitrilen zu erzielen und die Bildung von sekundären und tertiären Aminen zu vermeiden.

Die Hydrierung von Nitrilen erfolgt in der Regel durch katalytische Hydrierung an Edelmetallen, wie Pt, Pd oder Rhodium, oder Co und Ni-Katalysatoren (siehe beispielsweise "Amines, Aliphatic", Ullmann's Encyclopedia of Industrial Chemistry, Published Online : 15 JUN 2000, DOI: 10.1002/14356007.a02_001).

Das Verfahren wird üblicherweise in Suspensionfahrweise oder in einem Festbettreaktor durchgeführt.

Bei der Suspensionsfahrweise muss der eingesetzte Katalysator vom Reaktionsgemisch abgetrennt werden, um ein wirtschaftliches Verfahren zu ermöglichen. Die Abtrennung ist mit verfahrenstechnischem Aufwand verbunden.

Bei der Verwendung von Katalysatoren auf Basis von Co, Ni oder Cu sind bei der Hydrierung im Festbett in der Regel sehr hohe Temperaturen und Drücke erforderlich, um die Bildung von sekundären und tertiären Aminen zu verringern, die durch Reaktion von primärem Amin mit partiell hydriertem Nitril (=Imin-Zwischenstufe) entstehen können.

Beispielsweise offenbart die EP-449089 die Hydrierung von Isophoronnitril zu Isophorondiamin bei 250 bar und in der WO 2007/128803 wird die Hydrierung von N,N-Dimethylaminopropionitril (DMAPN) zu N,N-Dimethylaminopropylamin (DMAPA) bei 180 bar beschrieben.

Diese drastischen Reaktionsbedingungen können die Bildung von anderen unerwünschten Nebenprodukten erhöhen und erfordern einen hohen Material- und Sicherheitstechnischen-Einsatz.

Die Aufgabe der vorliegenden Erfindung bestand darin ein Festbettverfahren für die Hydrierung von 3-(Dimethylamino)propionitril oder Isophoronnitrilimin und/oder Isophoronnitril zur Verfügung zu stellen, das den Einsatz von Hydrierkatalysatoren, insbesondere von Cu, Co und Ni-haltigen Katalysatoren, bei milderen Reaktionsbedingungen, d.h. insbesondere geringeren Drücken und/oder Temperaturen, ermöglicht. Ein weiteres Ziel der vorliegenden Erfindung bestand in der Bereitstellung eines Festbettverfahrens, bei dem hohe Ausbeuten und Selektivitäten bei der Nitrilhydrierung erzielt werden können und das zudem wirtschaftlich zu realisieren ist.

Insbesondere sollte die Bildung von sekundären und tertiären Aminen verringert werden, wie sie beispielsweise durch Reaktion von nicht umgesetzten Amin mit partiell hydriertem Nitril (=Imin-Zwischenstufe) gemäß Schema 1 entstehen können.

Schema 1:

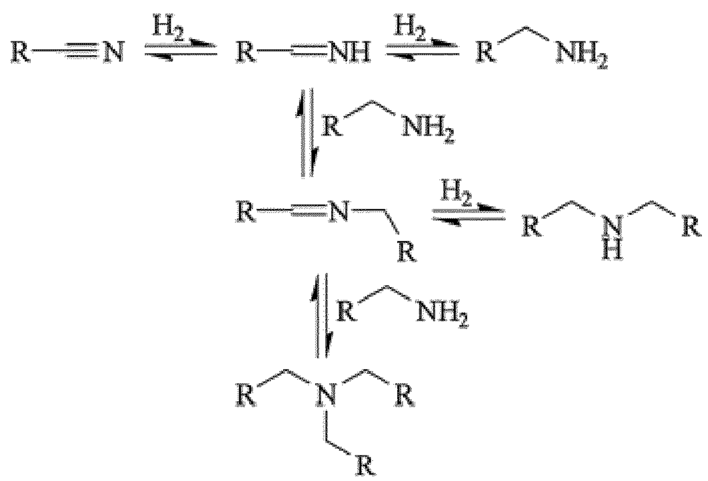


Erfindungsgemäß wurde die Aufgabe durch ein kontinuierliches Verfahren zur Hydrierung von Nitrilen mit Wasserstoff in Gegenwart eines Katalysators in einem Reaktor, wobei der Katalysator in einem Festbett angeordnet ist,

dadurch gekennzeichnet, dass die Querschnittsbelastung im Reaktor im Bereich von 5 kg/($m^2$ s) bis 50 kg/($m^2$ s) liegt, gelöst.

In dem erfindungsgemäßen Verfahren werden 3-(Dimethylamino)propionitril oder Isophoronnitrilimin und/oder Isophoronnitril hydriert.

Besonders bevorzugte cyclische Nitrile sind Isophoronnitrilimin (IPNI) und oder Isophoronnitril (IPN) zur Herstellung von Isophorondiamin und Isophthalodinitril zur Herstellung von meta-Xylylendiamin.

In einer weiteren besonders bevorzugten Ausführungsform wird Isophoronnitrilimin zur Herstellung von Isophorondiamin in das erfindungsgemäße Verfahren eingesetzt

Als Reduktionsmittel können Wasserstoff oder ein Wasserstoff enthaltendes Gas verwendet werden. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Bei dem erfindungsgemäßen Verfahren zur Herstellung von Aminen durch Reduktion von Nitrilen, kann die Hydrierung ggf. unter Zusatz von Ammoniak erfolgen. Bevorzugt wird in das Verfahren reiner Ammoniak eingesetzt, bevorzugt Ammoniak mit einem Gehalt von mehr als 99 Gew.-% Ammoniak und besonders bevorzugt mehr als 99,9 Gew.-% Ammoniak

Als Katalysatoren zur Hydrierung der Nitril-Funktion zum korrespondierenden Amin können insbesondere Katalysatoren eingesetzt werden, die als aktive Komponente ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Eine weitere bevorzugte aktive Komponente ist Cu.

Die oben genannten Katalysatoren können in üblicher Weise mit Promotoren, beispielsweise mit Chrom, Eisen, Kobalt, Mangan, Molybdän, Titan, Zinn, Metallen der Alkaligruppe, Metallen der Erdalkaligruppe und/oder Phosphor dotiert werden.

Als Katalysatoren können bevorzugt sogenannte Skelett-Katalysatoren (auch als Raney®-Typ bezeichnet, nachfolgend auch: Raney-Katalysator) eingesetzt werden, die durch Auslaugen (Aktivierung) einer Legierung aus hydrieraktivem Metall und einer weiteren Komponente (bevorzugt Al) erhalten werden. Bevorzugt werden Raney-Nickel-Katalysatoren oder Raney-Cobalt-Katalysatoren eingesetzt.

Als Katalysatoren werden weiterhin bevorzugt Pd oder Pt-Trägerkatalysatoren eingesetzt. Bevorzugte Trägermaterialien sind Aktivkohle, $Al_2O_3$, $TiO_2$, $ZrO_2$ und $SiO_2$.

In einer ganz bevorzugten Ausführungsform werden in das erfindungsgemäße Verfahren Katalysatoren eingesetzt, die durch Reduktion von sogenannten Katalysatorvorläufern hergestellt werden.

Der Katalysatorvorläufer enthält eine aktive Masse, die eine oder mehrere katalytisch aktive Komponenten, ggf. Promotoren und optional ein Trägermaterial enthält.

Bei den katalytisch aktiven Komponenten handelt es sich um sauerstoffhaltige Verbindungen der oben genannten Metalle, beispielsweise um deren Metalloxide bzw. Hydroxide, wie CoO, NiO, CuO und/oder deren Mischoxide.

Im Rahmen dieser Anmeldung wird der Begriff katalytisch aktive Komponenten für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Besonders bevorzugt sind Katalysatorvorläufer, wie

die in EP-A-0636409 offenbarten Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,2 bis 5,0 Gew.-% Alkali, berechnet als $M_2O$ (M=Alkali), enthalten, oder
in EP-A-0742045 offenbarte Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,05 bis 5 Gew.-% Alkali, berechnet als $M_2O$ (M=Alkali), enthalten, oder
in EP-A-696572 offenbarte Oxidgemische, die vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$, enthält, beispielsweise der in loc. cit, Seite 8, offenbarte Katalysator mit der Zusammensetzung 31,5 Gew.-%

$ZrO_2$, 50 Gew.-% NiO, 17 Gew.-% CuO und 1,5 Gew.-% $MoO_3$, enthalten oder
in EP-A-963 975 offenbarte Oxidgemische, die vor der Reduktion mit Wasserstoff 22 bis 40 Gew.-% $ZrO_2$, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO, enthalten.

Die Katalysatoren bzw. Katalysatorvorläufer werden bevorzugt in Form von Formkörpern in das erfindungsgemäße Verfahren eingesetzt.

Als Formkörper eignen sich Formkörper mit beliebiger Geometrie bzw. Form. Bevorzugte Formen sind Tabletten, Ringe, Zylinder, Sternstränge, Wagenräder oder Kugeln, besonders bevorzugt sind Tabletten, Ringe, Zylinder, Kugeln oder Sternstränge. Ganz besonders bevorzugt ist die Strangform.

Bei Kugeln beträgt der Durchmesser der Kugelform bevorzugt 10 mm oder weniger, besonders bevorzugt 4 mm oder weniger, ganz besonders bevorzugt 3 mm oder weniger und insbesondere bevorzugt 2,5 mm oder weniger.
In einer bevorzugten Ausführungsform liegt bei Kugeln der Durchmesser der Kugelform bevorzugt im Bereich von 0,1 bis 10, besonders bevorzugt 0,5 bis 4 mm, ganz besonders bevorzugt 1 bis 3 mm und insbesondere besonders bevorzugt 1,5 bis 2,5 mm.

Bei Strängen oder Zylindern liegt das Verhältnis von Länge : Durchmessers bevorzugt im Bereich von 1:1 bis 20:1, besonders bevorzugt 1:1 bis 14:1, ganz besonders bevorzugt im Bereich von 1:1 bis 10:1 und insbesondere bevorzugt im Bereich von 1:2 bis 6:1.
Der Durchmesser der Stränge oder Zylinder beträgt bevorzugt 10 mm oder weniger, besonders bevorzugt 5 mm oder weniger, ganz besonders bevorzugt 3 mm oder weniger und insbesondere bevorzugt 2,5 mm oder weniger.
In einer bevorzugten Ausführungsform liegt der Durchmesser der Stränge oder Zylinder vorzugsweise im Bereich von 0,1 bis 10 mm, besonders bevorzugt im Bereich von 0,5 bis 3 mm, ganz besonders bevorzugt im Bereich von 1 bis 2,5 mm und insbesondere bevorzugt im Bereich von 1,5 bis 2,5 mm.

Bei Tabletten beträgt die Höhe h der Tablette vorzugsweise 10 mm oder weniger, besonders bevorzugt 4 mm oder weniger, ganz besonders bevorzugt 3 mm oder weniger und insbesondere bevorzugt 2,5 mm oder weniger.
In einer bevorzugten Ausfürhungsform liegt die Höhe h der Tablette bevorzugt im Bereich von 0,1 bis 10 mm, besonders bevorzugt im Bereich von 0,5 bis 4 mm, ganz besonders bevorzugt im Bereich von 1 bis 3 mm und insbesondere bevorzugt im Bereich von 1,5 bis 2,5 mm.
Das Verhältnis von Höhe h (bzw. Dicke) der Tablette zum Durchmesser D der Tablette beträgt bevorzugt 1:1 bis 1:5, besonders bevorzugt 1:1 bis 1:2,5, ganz besonders bevorzugt 1:1 bis 1:2 und insbesondere bevorzugt 1:1 bis 1:2.

Bei allen anderen Geometrien weist der Katalysatorformkörper im erfindungsgemäßen Verfahren jeweils bevorzugt einen Äquivalentdurchmesser L = 1/a' von 2 mm oder weniger, besonders bevorzugt 1 mm oder weniger, ganz besonders bevorzugt 0,7 mm oder weniger und insbesondere bevorzugt 0,5 mm oder weniger auf, wobei a' die externe Oberfläche per Volumeneinheit ($mms^2/mmp^3$) ist, mit:

$$a' = \frac{A_p}{V_p},$$

wobei $A_p$ die externe Oberfläche des Formkörpers ($mm_s^2$) und $V_p$ das Volumen des Formkörpers ($mmp^3$) ist.
In einer bevorzugten Ausfürhungsform weist der Katalysatorformkörper im erfindungsgemäßen Verfahren bei allen anderen Geometrien jeweils bevorzugt einen Äquivalentdurchmesser L = 1/a' im Bereich von 0,1 bis 2mm, besonders bevorzugt im Bereich von 0,1 bis 0,7 mm, ganz besonders bevorzugt im Bereich von 0,2 bis 0,5 mm und insbesondere bevorzugt im Bereich von 0,3 bis 0,4 mm auf.

Die Oberfläche und das Volumen des Formkörpers ergeben sich aus den geometrischen Abmessungen des Formkörpers gemäß den bekannten mathematischen Formeln.

Das Volumen kann auch nach folgender Methode berechnet werden, bei der man:

1. Die innere Porosität des Formkörpers bestimmt (z.B. über Messung der Wasseraufnahme in [ml/g Kat] bei Raumtemperatur und 1 bar Gesamtdruck),
2. die Verdrängung des Formkörpers beim Eintauchen in eine Flüssigkeit (z.B. durch Gasverdrängung mittels Helium-Pyknometer) bestimmt und

3. die Summe beider Volumina bildet.

Die Oberfläche kann auch nach folgender Methode theoretisch berechnet werden, bei der man eine Umhüllende des Formkörpers definiert, deren Kurvenradien max. 5 μm beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Der eingesetzte Formkörper weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l

In einer bevorzugten Ausführungsform werden die Katalysatoren in Form von Formkörpern in das erfindungsgemäße Verfahren eingesetzt, die durch Tränkung (Imprägnierung) von Trägermaterialien hergestellt werden, die oben genannte Geometrie aufweisen oder die nach der Tränkung zur Formkörpern, die die oben genannte Geometrie aufweisen verformt werden.

Als Trägermaterialien kommen beispielsweise Kohlenstoff, wie Graphit, Ruß, Graphen, Kohlenstoffnanoröhrchen und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800°C, vorzugsweise 350 bis 600°C, insbesondere bei 450 bis 550°C ausgeführt.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Bevorzugt werden Trägermaterialien eingesetzt, die bereits die zu vor beschriebene bevorzugte Geometrie der Formkörper aufweisen.

Es ist jedoch auch möglich Trägermaterialien einzusetzen, die als Pulver oder Splitt vorliegen, und getränkten Trägermaterialien einer Formgebung zu unterziehen.

So kann beispielsweise das imprägnierte und getrocknete bzw. calcinierten Trägermaterial konditioniert werden.

Die Konditionierung kann beispielsweise dadurch erfolgen, dass man das getränkte Trägermaterial durch Vermahlen auf eine bestimmte Korngröße einstellt.

Nach der Vermahlung kann das konditioniete, getränkte Trägermaterial mit Formhilfsmitteln wie Graphit, oder Stearinsäure vermischt werden und zu Formkörpern weiterverarbeitet werden. Gängige Verfahren der Formgebung sind beispielsweise im Ullmann [Ullmann's Encyclopedia Electronic Release 2000, Kapitel: "Catalysis and Catalysts", Seiten 28-32] und von Ertl et al.

[Ertl, Knözinger, Weitkamp, Handbook of Heterogenoeous Catalysis, VCH Weinheim, 1997, Seiten 98 ff] beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

In einer bevorzugten Ausführungsform werden Formkörper in das erfindungsgemäße Verfahren eingesetzt, die durch eine gemeinsame Fällung (Mischfällung) aller ihrer Komponenten hergestellt werden und die so ausgefällten Katalysatorvorläufer einer Formgebung unterzogen werden.

Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.
Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

In einer weiteren, bevorzugten Ausführungsform können die Formkörper durch Auffällung hergestellt werden. Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.
Als Flüssigkeit, in der das Trägermaterial suspendiert wird, wird üblicherweise Wasser eingesetzt.
Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.
Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.
Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.
Die Konditionierung kann beispielsweise dadurch erfolgen, dass man den Fällungskatalysator durch Vermahlen auf eine bestimmte Korngröße einstellt.
Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al.
[Ertl, Knözinger, Weitkamp, Handbook of Heterogenoeous Catalysis, VCH Weinheim, 1997, Seiten 98 ff] beschrieben.
Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.
Durch den Prozess der Formgebung können Formkörper mit der oben genannten Geometrie erhalten werden.
Nach der Konditionierung bzw. Formgebung erfolgt in der Regel eine Temperung. Die Temperaturen bei der Temperung entsprechen üblicherweise den Temperaturen bei der Calcinierung.

Formkörper, die die durch Tränkung oder Fällung hergestellt wurden, enthalten in der Regel die katalytisch aktiven Komponenten nach erfolgter Calcinierung in der Regel in Form ihrer sauerstoffhaltige Verbindungen, beispielsweise um deren Metalloxide bzw. Hydroxide, wie CoO, NiO, CuO und/oder deren Mischoxide (Katalysatorvorläufer).

Die Katalysatorvorläufer, die wie voranstehend beschrieben durch Imprägnierung oder Fällung hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.

Als Reduktionsmittel wird üblicherweise Wasserstoff oder ein Wasserstoff enthaltendes Gas eingesetzt.
Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit FrischWasserstoff und ggf. nach Entfernen von Wasser durch Kondensation.
Die Reduktion des Katalysatorvorläufers erfolgt bevorzugt in einem Reaktor, in dem die Formkörper als Festbett angeordnet sind. Besonders bevorzugt erfolgt die Reduktion des Katalysatorvorläufers in demselben Reaktor in dem die nachfolgende Umsetzung der Nitrile mit Wasserstoff erfolgt.
Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.
Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.
Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der so erhaltene Formkörper kann nach der Reduktion unter inerten Bedingungen gehandhabt werden. Bevorzugt kann der Formkörper unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.
Die Lagerung des Katalysators unter inerten Substanzen ermöglicht eine unkomplizierte und ungefährliche Handhabung und Lagerung des Formkörpers.

Der Formkörper kann nach der Reduktion aber auch mit einem Sauerstoff enthaltenden Gasstrom wie Luft oder einem Gemisch von Luft mit Stickstoff in Kontakt gebracht werden. Dadurch wird ein passivierter Formkörper erhalten. Der passivierte Formkörper weist im Allgemeinen eine schützende Oxidschicht auf. Durch diese schützende Oxidschicht wird die Handhabung und Lagerung des Katalysators vereinfacht, so dass beispielsweise der Einbau des passivierten Formkörpers in den Reaktor vereinfacht wird. Ein passivierter Formkörper wird bevorzugt vor dem Inkontakt bringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

Das erfindungsgemäße Verfahren wird bevorzugt in einem Reaktor durchgeführt, in dem der Katalysator als Festbett angeordnet ist.

In einer bevorzugten Ausführungsform umfasst die Festbettanordnung eine Katalysatorschüttung im eigentlichen Sinn, d. h. lose, geträgerten oder ungeträgerte Formkörper, die bevorzugt in der zuvor beschriebenen Geometrie oder Form vorliegen.

Dazu werden die Formkörper in den Reaktor eingebracht.
Damit die Formkörper in dem Reaktor verbleiben und nicht durch diesen hindurchfallen, wird üblicherweise ein Gitterboden oder ein gas- und flüssigkeitsdurchlässiges Blech eingesetzt, auf dem die Formkörper aufliegen.

Die Formkörper können sowohl am Eingang als am Ausgang des Reaktors von einem Inertmaterial umgeben sein. Als Inertmaterial werden in der Regel Formkörper eingesetzt, die eine ähnliche Geometrie aufweisen, wie die zuvor beschriebenen Katalysatorformköprer, sich jedoch in der Reaktion inert verhalten, z.B. Pallringe, Kugeln aus einem inerten Material (z.B. Keramik, Steatit, Aluminium).
Die Formkörper können aber auch mit Inertmaterial durchmischt werden und als Mischung in den Reaktor eingebracht werden.

Die Katalysatorschüttung (Formkörper + ggf. Inertmaterial) weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l.

Der Differenzdruck über die Schüttung beträgt bevorzugt weniger als 1000 mbar/m, bevorzugt weniger als 800 mbar/m und besonders bevorzugt weniger als 700 mbar/m. Bevorzugt liegt der Differenzdruck über die Schüttung im Bereich von 10 bis 1000 mbar/m, bevorzugt 50 bis 800 mbar/m, besonders bevorzugt 100 bis 700 mbar/m und insbesondere im Bereich von 200 bis 500 mbar/m.
Bei Rieselfahrweise (Durchflussrichtung der Flüssigkeit von oben nach unten) ergibt sich der Differenzdruck aus dem

oberhalb der Katalysatorschüttung gemessenen Druck und dem unterhalb der Katalysatorschüttung gemessenen Druck. Bei Sumpffahrweise (Durchflussrichtung der Flüssigkeit von unten nach oben) ergibt sich der Differenzdruck aus dem unterhalb der Katalysatorschüttung gemessenen Druck und dem oberhalb der Katalysatorschüttung gemessenen Druck.

Geeignete Festbettreaktoren sind beispielsweise in dem Artikel "Fixed-Bed Reactors" (Ullmann's Encyclopedia of Industrial Chemistry, Published Online: 15 JUN 2000, DOI: 10.1002/14356007.b04_199) beschrieben.

Bevorzugt wird das Verfahren in einem Schachtreaktor, Rohrbündelreaktor oder Rohrreaktor durchgeführt.

Besonders bevorzugt wird das Verfahren in einem Rohrreaktor durchgeführt.

Die Reaktoren können jeweils als einzelner Reaktor, als Serie von einzelnen Reaktoren und/oder in Form von zwei oder mehr parallelen Reaktoren eingesetzt werden.

Der spezielle Reaktoraufbau und die Durchführung der Reaktion können in Abhängigkeit von dem durchzuführenden Hydrierungsverfahren, den erforderlichen Reaktionszeiten und der Natur des eingesetzten Katalysators variieren.

Bevorzugt beträgt das Verhältnis von Höhe zu Durchmesser des Reaktors, insbesondere eines Rohrreaktors, 1:1 bis 500:1, besonders bevorzugt 2:1 bis 100:1 und insbesondere bevorzugt 5:1 bis 50:1.

Die Fließrichtung der Reaktanden (Edukte, Wasserstoff, ggf. flüssiger Ammoniak) ist in der Regel von oben nach unten bzw. von unten nach oben.
Besonders bevorzugt ist die Fließrichtung der Reaktanden (Edukte, Wasserstoff, ggf. flüssiger Ammoniak) von oben nach unten durch den Reaktor.

Die Katalysatorbelastung bei kontinuierlicher Fahrweise liegt typischerweise bei 0,01 bis 10, vorzugsweise von 0,2 bis 5, besonders bevorzugt von 0,2 bis 4 kg Edukt pro L Katalysator und Stunde.

Die Querschnittsbelastung liegt erfindungsgemäß im Bereich von 5 kg/($m^2$ s) bis 50 kg/($m^2$ s), bevorzugt 8 bis 25 kg/($m^2$ s), besonders bevorzugt 10 bis 20 kg/($m^2$ s) und insbesondere bevorzugt 12 bis 18 kg/($m^2$ s).

Die Querschnittsbelastung v [kg/($m^2$ s)] ist definiert als

$$v = \frac{Q}{A},$$

wobei Q die Durchflussmasse [kg/s] und A die Querschittsfläche der leeren Kolonne ist [$m^2$]. Die Durchflussmasse Q ist wiederum definiert als die Summe der Massen aller zugeführten Eduktströme und Rückführströme. Wasserstoff, Kreisgase und eventuell zugeführte Inertgase werden nicht zur Berechnung der Durchflussmasse verwendet, da Wasserstoff, Kreisgase und Inertgase bei den üblichen Hydrierbedingungen in der Regel in der Gasphase vorliegen.

Um die hohen Querschnittsbelastungen zu erzielen, wird bevorzugt ein Teil des Austrags (Teilaustrag) aus dem Hydrierreaktor als Rückführstrom in den Reaktor zurückgeführt (Umlaufstrom). Der Umlaufstrom kann dem Reaktor separat zugeführt werden oder er kann besonders bevorzugt mit den zugeführten Edukten vermischt werden und zusammen mit diesem dem Reaktor wieder zugeführt werden.
Das Verhältnis von Umlaufstrom zum zugeführten Eduktstrom liegt bevorzugt im Bereich von 0,5:1 bis 250:1, besonders bevorzugt im Bereich von 1:1 bis 200:1, und insbesondere bevorzugt im Bereich von 2:1 bis 180:1. Wenn in das Verfahren kein Ammoniak zugeführt wird, dann liegt das Verhältnis von Umlaufstrom zum zugeführten Eduktstrom bevorzugt im oberen Bereich der voranstehend genannten Bereiche. Wenn hingegen in das Verfahren viel Ammoniak zugeführt wird, dann liegt das Verhältnis von Umlaufstrom zum zugeführten Eduktstrom bevorzugt im unteren Bereich der voranstehend genannten Bereiche.

In einer weiteren bevorzugten Ausführungsform können hohe Querschnittsbelastungen erzielt werden, wenn die Reaktion in einem Reaktor mit schlanker Bauweise, insbesondere in einem Rohrreaktor mit schlanker Bauweise, durchgeführt wird.
Das Verhältnis von Höhe zu Durchmesser des Reaktors liegt deshalb, wie voranstehend beschrieben, bevorzugt im Bereich von 1:1 bis 500:1, besonders bevorzugt im Bereich von 2:1 bis 100:1 und insbesondere bevorzugt im Bereich von 5:1 bis 50:1.

Die Hydrierung wird in der Regel bei einem Druck von 1 bis 200 bar, insbesondere von 5 bis 150 bar, bevorzugt von 10 bis 100 bar und besonders bevorzugt von 15 bis 95 bar durchgeführt. Ganz besonders bevorzugt wird die Hydrierung bei einem Druck von weniger als 95 bar als Niederdruckverfahren ausgeführt.

Die Temperatur liegt in der Regel in einem Bereich 25 bis 300°C, insbesondere von 50 bis 200°C, bevorzugt von 70 bis 150°C, besonders bevorzugt von 80 bis 140°C.

Dabei werden die Reaktionsbedingungen bevorzugt so gewählt, dass die eingesetzten Nitrile und ggf. zugegebene Flüssigkeiten sowie ggf. zugeführter Ammoniak in der Regel in der Flüssigphase vorliegen und nur der eingesetzte Wasserstoff bzw. Inertgase unter den genannten Reaktionsbedingungen in der Gasphase vorliegen.

Das molare Verhältnis von Wasserstoff zu eingesetzten Nitril beträgt in der Regel 2:1 bis 25:1, vorzugsweise

2,01:1 bis 10:1. Der Wasserstoff kann als Kreisgas in die Reaktion zurückgeführt werden.

Bei dem erfindungsgemäßen Verfahren zur Herstellung von Aminen durch Reduktion von Nitrilen, kann die Hydrierung unter Zusatz von Ammoniak erfolgen. Ammoniak wird in der Regel dabei in Molverhältnissen zur Nitrilgruppe im Verhältnis von 0,5 : 1 bis 100 : 1, vorzugsweise 2 : 1 bis 20 : 1 eingesetzt. Die bevorzugte Ausführungsform ist jedoch ein Verfahren, bei dem kein Ammoniak zugegeben wird.

Die Reaktion kann in Substanz oder in einer Flüssigkeit durchgeführt werden.
Die Hydrierung erfolgt bevorzugt in Gegenwart einer Flüssigkeit.
Geeignete Flüssigkeiten sind beispielsweise C1- bis C4-Alkohole, wie Methanol oder Ethanol, C4- bis C12-Dialkylether, wie Diethylether oder tert-Butylmethylether, oder cyclische C4- bis C12-Ether, wie Tetrahydrofuran oder Dioxan, oder Kohlenwasserstoffe, wie Pentan, Hexan, Heptan, Octan, Cyclohexan oder Toluol. Geeignete Flüssigkeiten können auch Mischungen der vorstehend genannten Flüssigkeiten sein. In einer bevorzugten Ausführungsform ist die Flüssigkeit ein Produkt der Hydrierung.

Die Reaktion kann auch in Gegenwart von Wasser erfolgen. Der Wassergehalt sollte allerdings nicht mehr als 10 Gew.-%, vorzugsweise weniger als 5 Gew.-%, besonders bevorzugt weniger als 3 Gew.-% betragen, bezogen auf die Masse der eingesetzten Flüssigkeit, um das Auslaugen und/oder Abwaschen der Verbindungen der Alkali-, Erdalkali- und/oder Seltenen Erdmetalle weitestgehend zu vermeiden.

Die Aktivität und/oder Selektivität der erfindungsgemäßen Katalysatoren kann mit zunehmender Standzeit abnehmen. Demgemäß wurde ein Verfahren zur Regenerierung der erfindungsgemäßen Katalysatoren gefunden, bei dem man den Katalysator mit einer Flüssigkeit behandelt. Die Behandlung des Katalysators mit einer Flüssigkeit soll dazu führen, dass eventuell anhaftende Verbindungen, die aktive Stellen des Katalysators blockieren, abgelöst werden. Die Behandlung des Katalysators mit einer Flüssigkeit kann durch Rühren des Katalysators in einer Flüssigkeit oder durch Waschen des Katalysators in der Flüssigkeit erfolgen, wobei nach erfolgter Behandlung die Flüssigkeit durch Filtration oder Abdekantieren zusammen mit den abgelösten Verunreinigungen vom Katalysator abgetrennt werden kann.

Geeignete Flüssigkeiten sind in der Regel das Produkt der Hydrierung, Wasser oder ein organisches Lösungsmittel, bevorzugt Ether, Alkohole oder Amide.

In einer weiteren Ausführungsform kann die Behandlung des Katalysators mit Flüssigkeit in Gegenwart von Wasserstoff oder eines Wasserstoff enthaltenden Gases erfolgen.

Diese Regenerierung kann unter erhöhter Temperatur, in der Regel von 20 bis 250°C, durchgeführt werden. Es ist auch möglich, den gebrauchten Katalysator zu trocknen und anhaftende organische Verbindungen mit Luft zu flüchtigen Verbindungen wie $CO_2$ zu oxidieren. Vor einer weiteren Verwendung des Katalysators in der Hydrierung muss dieser nach erfolgter Oxidation in der Regel, wie zuvor beschrieben aktiviert werden.

Bei der Regenerierung kann der Katalysator mit einer löslichen Verbindung der katalytisch aktiven Komponenten in Kontakt gebracht werden. Das Inkontaktbringen kann in der Art erfolgen, dass der Katalysator mit einer wasserlöslichen Verbindung der katalytisch aktiven Komponente getränkt oder benetzt wird.

Bei der Hydrierung von Nitrilen zu den entsprechenden Aminen ist es häufig erforderlich einen hohen Umsetzungsgrad bezüglich der eingesetzten Nitrile zu erzielen, da nicht umgesetzte oder nur teilweise umgesetzte Nitrile nur schwer abzutrennen sind und in den Folgeanwendungen zu unerwünschten Eigenschaften, wie Geruch und Verfärbung, führen können.
Der Vorteil der vorliegenden Erfindung besteht darin, dass das erfindungsgemäße Verfahren die Hydrierung von Nitrilen in hoher Selektivität und Ausbeute ermöglicht. Außerdem wird die Bildung von unerwünschten Nebenprodukten verringert.

Dadurch ist es möglich, die Hydrierung unter milderen Reaktionsbedingungen, insbesondere bei niedrigerem Druck und/oder bei niedrigerer Temperatur durchzuführen.
Somit ermöglich die vorliegende Erfindung ein wirtschaftliches Verfahren zur Hydrierung. Insbesondere wird die Bildung von sekundären und tertiären Aminen, wie sie beispielsweise durch Reaktion von nicht umgesetzten Amin mit partiell hydriertem Nitril (=Imin-Zwischenstufe) gemäß Schema 1 entstehen kann, verringert.

Insbesondere ermöglicht das erfindungsgemäße Verfahren die Herstellung von Isophorondiamin mit hoher Selektivität und Ausbeute. Insbesondere ist es möglich den Gehalt an unerwünschtem Isophoronnitrilamin (IPNA) zu verringern. IPNA kann beispielsweise durch Reaktion von Isophoronnitril mit Ammoniak entstehen, welches zunächst zum Isophoronntrilimin reagiert, welches dann bevorzugt mit Wasserstoff zum Isophoronnitrilamin reagiert.

Isophorondiamin dient als Zwischenprodukt zur Herstellung von Härtern für Epoxidharze und Beschichtungen (z.B. 3-isocyanatomethyl-3,5,5-trimethylcyclohexyl-isocyanat) und wird selbst auch direkt als Härter eingesetzt. Weitere Anwendungen sind Beschichtungen mit hervorragenden Korrosionsschutzeigenschaften für Metalle und Klebstoffverbindungen. Es wird ferner bei der Herstellung von nicht-kristallinen Spezialpolyamiden, als Kettenverlängerer bei Polyurethanen und als Zwischenprodukt zur Herstellung von Farbstoffen verwendet.
Somit betrifft die vorliegende Erfindung auch ein Verfahren zur Herstellung von Härtern für Epoxidharze und Beschichtungen, Spezialpolyamiden, Polyurethanen und Farbstoffen, dadurch gekennzeichnet, dass man in einer ersten Stufe Isophorondiamin aus Isophoronnitrilimin gemäß Anspruch 1 hergestellt und das in der ersten Stufe erhaltene Isophorondiamin in einer zweiten Stufe zur Herstellung von Härtern für Epoxidharze und Beschichtungen, Spezialpolyamiden, Polyurethanen und Farbstoffen einsetzt.
Aufgrund des geringen Gehalts an IPNA können auch die Folgeprodukte vorteilhafte Eigenschaften aufweisen.

Das erfindungsgemäße Verfahren wird ebenfalls bevorzugt zur Herstellung von 3-(Dimethylamino)propylamin (DMAPA). Insbesondere ermöglicht das erfindungsgemäße Verfahren eine Verringerung des Gehalts an Bis-DMAPA. Als Zwischenprodukt wird es z.B. zur Herstellung von oberflächenaktiven Substanzen, Seifen, Kosmetik, Shampoos, Hygieneprodukten, Waschmittel und Pflanzenschutzmitteln verwendet. DMAPA wird auch zur Wasserbehandlung und als Polymerisationskatalysator für PU und Epoxy eingesetzt.

Somit betrifft die vorliegende Erfindung auch ein Verfahren zur Herstellung oberflächenaktiven Substanzen, Seifen, Kosmetik, Shampoos, Hygieneprodukten, Waschmittel und Pflanzenschutzmitteln, dadurch gekennzeichnet, dass man in einer ersten Stufe DMAPA aus 3-(Dimethylamino)propionitril gemäß Anspruch 1 hergestellt und das in der ersten Stufe erhaltene DMAPA in einer zweiten Stufe zur Herstellung von oberflächenaktiven Substanzen, Seifen, Kosmetik, Shampoos, Hygieneprodukten, Waschmittel und Pflanzenschutzmitteln einsetzt. Aufgrund des geringen Gehalts an Bis-DMAPA können auch die Folgeprodukte vorteilhafte Eigenschaften aufweisen.

Die Erfindung wird anhand der folgenden Beispiele erläutert:

Beispiele:

Definitionen:

Die Katalysatorbelastung wird angegeben als der Quotient von Eduktmasse im Zulauf und dem Produkt aus Katalysatorvolumen und Zeit.
Katalysatorbelastung = Eduktmasse/(Volumen des Katalysators · Reaktionszeit).
Die Einheit der Katalysatorbelastung wird in [$kg_{Edukt}$/(l·h)] angegeben.

Die angegebenen Selektivitäten wurden durch gaschromatographische Analysen bestimmt und aus den Flächenprozenten errechnet.

GC-Programme:

IPDA: GC-Säule: 60 m DB1701; ID = 0,32 mm, Filmdicke = 0,25μm
Temperaturprogramm: 60 °C - 5 °C/min - 280 °C - 20 min
DMAPA: GC-Säule: 60 m CP Volamnin; WCOT Fused Silica 0,32 mm
Temperaturprogramm: 50 °C - 10 min - 15 °C/min - 240 °C - 30 min

Der Edukt-Umsatz U(E) berechnet sich nach folgender Formel:

$$U(E) = \frac{F\%(E)_{Anfang} - F\%(E)_{Ende}}{F\%(E)_{Anfang}}$$

Die Ausbeute an Produkt A(P) ergibt sich aus den Flächenprozenten des Produktsignals.

$$A(P) = F\%(P),$$

wobei die Flächenprozente F%(i) eines Eduktes (F%(E)), Produktes (F%(P)), eines Nebenproduktes (F%(N)) oder ganz allgemein eines Stoffes i (F%(i)), sich aus dem Quotient der Fläche F(i) unterhalb des Signals des Stoffes i und der Gesamtfläche
$F_{Gesamt}$, d.h. die Summe der Fläche unterhalb der Signale i, multipliziert mit 100 ergeben:

$$F\%(i) = \frac{F(i)}{F_{Gesamt}} \cdot 100 = \frac{F(i)}{\sum_i F(i)} \cdot 100$$

Die Selektivität des Eduktes S(E) berechnet sich als Quotient von Produkt-Ausbeute A(P) und Edukt-Umsatz U(E):

$$S(E) = \frac{A(P)}{U(E)} * 100$$

Herstellung des Katalysators

Als Katalysator wurde ein Cobaltkatalysators mit einem Strangdurchmesser von 2 mm eingesetzt, dessen Herstellung in der EP-A-0636409 beschrieben ist (Beispiel Katalysator A).

Beispiel 1: Umsetzung von IPN zu IPDA

Die Reaktion wurde in zwei kontinuierlich betriebenen, hintereinander geschalteten Rohrreaktoren durchgeführt. Dabei wurde die Iminierung des Isophoronnitrils (IPN) mit Ammoniak zum Isophoronnitrilimin (IPNI) im ersten Reaktor bei 60°C an $TiO_2$ (75 ml) durchgeführt. Die Zulaufmenge an IPN betrug 84 g/h, die $NH_3$-Menge 180 g/h. Der Austrag des Iminierreaktors wurde zusammen mit Wasserstoff auf den zweiten Reaktor geleitet. Die Temperatur des zweiten Reaktors wurde auf 90°C eingestellt. Die zugeführte Wasserstoffmenge betrug 88 L/h. Als Katalysator wurden 180 ml des zuvor aktivierten Cobaltkatalysators eingesetzt. Der Hydrierreaktor hat einen Innen-Querschnitt von 16 mm, wobei im Reaktor eine Thermohülse mit einem Außendurchmesser von 3,7 mm eingebaut ist. Es wurde der Anteil an Aminonitril (IPNA) im Reaktoraustrag des Hydrierreaktors bei verschiedenen Rückführströmen bestimmt (siehe Tabelle 1). Die Angabe der Flüssigkeitsrückführung bezieht sich auf das Verhältnis des Rückführstroms zur Summe zugeführten IPNs und Ammoniaks. In *Tabelle* 1 sind IPNA- und IPDA-Werte bei verschiedenen Rücklauf:Zulaufverhältnissen und Drücken angegeben. Dazu wurde die Querschnittsbelastung (QB) für jede der Einstellungen berechnet. Die Prozentangaben sind GC-Fl.%.

Tabelle 1: Werte nach der Hydrierung

| Druck | RL:ZL | IPNA | IPDA | Querschnittsbelastung |
|---|---|---|---|---|
| 80 bar | 10:1 | 24 % | 62 % | 4,2 $kg/m^2/s$ |
| 80 bar | 20:1 | 8 % | 85 % | 8,1 $kg/m^2/s$ |
| 80 bar | 40:1 | 7 % | 87 % | 15,8 $kg/m^2/s$ |
| 70 bar | 10:1 | 19 % | 73 % | 4,2 $kg/m^2/s$ |
| 70 bar | 40:1 | 7 % | 85 % | 15,8 $kg/m^2/s$ |

Aus der Tabelle wird ersichtlich, dass bei höheren Rückführströmen der Anteil an IPNA und damit an teilhydriertem Produkt überraschend abnimmt, während der Anteil an IPDA zunimmt. Überraschend ist der Befund insofern, als man bei sehr hohen Rückführraten eine Rührkesselcharakteristik des Rohrreaktors erwarten würde. Dadurch sollte der Anteil an nicht hydrierten Komponenten im Austrag ansteigen. So wird bei 80 bar durch Erhöhung der Rückführmenge und damit einer Erhöhung der Querschnittsbelastung der IPNA-Anteil von zunächst 24% bei 4,2 $kg/m^2/s$ bis auf 7% bei 15,8 $kg/m^2/s$ vermindert. Gleichzeitig steigt der IPDA-Anteil entsprechend an (62% bzw. 87%). Auch bei 70 bar ist dieser Effekt zu beobachten: hier vermindert sich der IPNA-Anteil von zunächst 19% bei einer QB von 4,2 $kg/m^2/s$ auf 7% bei einer QB von15,8 $kg/m^2/s$, der IPDA-Anteil steigt von 73% auf 85%.

Beispiel 2: Umsetzung von DMAPN zu DMAPA

Die Reaktion wurde in einem Rohrreaktor (Innendurchmesser 0,6 cm, Länge 1 m) mit Flüssigkeitsrückführung bei 90°C und 85 bar in Gegenwart von Wasserstoff und Ammoniak durchgeführt. Der Reaktor wurde mit 40,2 g Cobaltkatalysator befüllt. Vor Reaktionsbeginn wurde der reduziert passivierte Kobalt-Katalysator 12 Stunden bei 280°C (1 bar) im Wasserstoffstrom reduziert und mit DMAPA angefahren. Anschließend wurde der Reaktor auf die gewünschte Reaktionstemperatur abgekühlt und 85 bar Wasserstoff aufgepresst. Vor Umstellung des Zulaufs auf DMAPN wurden die Flüssigkeitsrückführung und die Ammoniakdosierung eingestellt.

Die Zulaufmenge an DMAPN betrug zunächst 19.4 g/h, die $NH_3$-Menge 19 g/h. Die zugeführte Wasserstoffmenge betrug 25 L/h Es wurde eine Flüssigkeitsrückführung des Hydrieraustrags von 40 g/h eingestellt (vgl. 1) Tabelle 2). Durch Erhöhung der zurückgeführten Flüssigkeitsmenge und Erhöhung des Rücklaufverhältnisses (RL:ZL) wurde bei ansonsten gleichbleibenden Reaktionsbedingungen die Querschnittsbelastung (QB) um den Faktor 10 erhöht. Durch die erhöhte QB wurde die unerwünschte Bildung von Bis-DMAPA vollständig unterdrückt (vgl. 2) Tabelle 2).

Tabelle 2: DMAPA

| | Druck [bar] | Temp. [°C] | Belastung [kg/l*h] | DMAPN [g/h] | RL:ZL [g/h:g/h] | QB [kg/m$^2$/s] | Umsatz [FI%] | Bis-DMAPA [FI%] | DMAPA [FI%] |
|---|---|---|---|---|---|---|---|---|---|
| 1) | 85 | 90 | 1,00 | 19,4 | 2,1 | 0,77 | 100,00 | 0,87 | 99,13 |
| 2) | 85 | 90 | 1,00 | 19,4 | 38,5 | 7,77 | 99,75 | 0,00 | 99,75 |

Beispiel 3: Umsetzung von DMAPN zu DMAPA

Die Reaktion wurde in einem von oben nach unten durchströmten Rohrreaktor (Innendurchmesser 0,5 cm, Länge 1 m, Querschnittsfläche 0,196 cm$^3$) mit Flüssigkeitsrückführung bei 90°C und 85 bar in Gegenwart von Wasserstoff durchgeführt. Vor Reaktionsbeginn wurden 24,5 g des reduziert-passivierten Kobalt-Katalysators (Herstellung: kommerziell bei Aldrich erhältliches Lithium-Cobalt(III)-oxid ("Lithiumcobaltit", $LiCoO_2$) wurde unter Zusatz von 25 % Zinkoxid-Pulver sowie verdünnter Salpetersäure, Polyethylenoxid und Acronal V312 zu 4 mm-Formkörper extrudiert; danach Reduktion bei 300 °C/1 bar im Wasserstoffstrom, danach Passivierung) in den Reaktor eingebaut und 12 Stunden bei 300 °C (1 bar) und im Wasserstoffstrom (25 Nl/h) aktiviert. Anschließend wurde der Reaktor auf die Anfangs-Reaktionstemperatur von 150 °C abgekühlt, 85 bar Wasserstoff aufgepresst und mit DMAPA angefahren. Es wurde ein Wasserstoffstrom von 50 Nl/h eingestellt. Es wurde eine Flüssigkeitsrückführung von 934 g/h eingestellt. Die Zulaufmenge an DMAPN betrug 6 g/h (entspr. einer Querschnittsbelastung (QB von 13,32 kg/m$^2$/s)). Der Umsatz an DMAPN lag bei 99 %, die Menge an gebildetem Bis-DMAPA lag bei 0,1 %. Anschließend wurde die Flüssigkeitsrückführung auf einen niedrigeren Wert von 63 g/h eingestellt. Dadurch wurde bei ansonsten gleichbleibenden Reaktionsbedingungen die QB um den Faktor 15 erniedrigt (entspr. QB von 0,98 kg/m$^2$/s). Bei einem gleich hohen Umsatz von 99 % ergaben sich nunmehr 0,8 % Bis-DMAPA im Reaktionsaustrag.

**Patentansprüche**

1. Verfahren zur Hydrierung von Nitrilen mit Wasserstoff in Gegenwart eines Katalysators in einem Reaktor, wobei der Katalysator in einem Festbett angeordnet ist, **dadurch gekennzeichnet, dass** die Querschnittsbelastung im Reaktor im Bereich von 5 kg/(m$^2$ s) bis 50 kg/(m$^2$s) liegt und als Nitrile 3-(Dimethylamino)propionitril oder Isophoronnitrilimin und / oder Isophoronnitril eingesetzt werden.

2. Verfahren nach Anspruch 1, **dadurch gekennzeichnet, dass** das Festbett eine Katalysatorschüttung aus losen, geträgerten oder ungeträgerten Formkörpern ist.

3. Verfahren nach mindestens einem der Ansprüche 1 bis 2, **dadurch gekennzeichnet, dass** der Formkörper in Form von Tabletten, Ringe, Zylinder, Kugeln oder Sternstränge eingesetzt wird.

4. Verfahren nach mindestens einem der Ansprüche 1 bis 3, **dadurch gekennzeichnet, dass** die Schüttdichte der Schüttung 0,1 bis 3 kg/l beträgt.

5. Verfahren nach mindestens einem der Ansprüche 1 bis 4, **dadurch gekennzeichnet, dass** der Katalysator Co oder Ni enthält.

6. Verfahren nach mindestens einem der Ansprüche 1 bis 5, **dadurch gekennzeichnet, dass** der Katalysator durch Reduktion von Katalysatorvorläufern hergestellt wird.

7. Verfahren nach mindestens einem der Ansprüche 1 bis 6, **dadurch gekennzeichnet, dass** ein Teil des Austrags (Teilaustrag) aus dem Hydrierreaktor als Rückführstrom in den Reaktor zurückgeführt (Umlaufstrom) und das Verhältnis von Umlaufstrom zum zugeführten Eduktstrom im Bereich von 0,5:1 bis 250:1 liegt.

8. Verfahren nach mindestens einem der Ansprüche 1 bis 7, **dadurch gekennzeichnet, dass** der Druck im Bereich von 15 bis 85 bar und/oder die Temperatur im Bereich von 70 bis 150°C liegt.

9. Verfahren nach mindestens einem der Ansprüche 1 bis 8, **dadurch gekennzeichnet, dass** das Verfahren in einem Schachtreaktor, Rohrreaktor oder Rohrbündelreaktor durchgeführt wird.

10. Verfahren nach Anspruch 9, **dadurch gekennzeichnet, dass** das Verhältnis von Höhe zu Durchmesser des Rohrreaktors 1:1 bis 500:1 beträgt.

11. Verfahren nach mindestens einem der Ansprüche 1 bis 10, **dadurch gekennzeichnet, dass** der Differenzdruck über die Katalysatorschüttung weniger als 1000 mbar/m beträgt.

12. Verfahren nach mindestens einem der Ansprüche 1 bis 11, **dadurch gekennzeichnet, dass** als Nitril Isophoronnitrilimin oder 3-(Dimethylamino)propionitril eingesetzt wird.

13. Verfahren nach mindestens einem der Ansprüche 1 bis 12, **dadurch gekennzeichnet, dass** als Nitril Isopharonnitrilimin eingesetzt wird und das durch die Hydrierung hergestellte Isopherandiamin in einer weiteren Verfahrensstufe zur Herstellung von Härtem für Epoxidharze und Beschichtungen, Spezialpolyamiden, Polyurethanen und Farbstoffen eingesetzt wird.

14. Verfahren nach mindestens einem der Ansprüche 1 bis 12, **dadurch gekennzeichnet, dass** als Nitril 3-(Dimethylamino)propionitril eingesetzt wird und das durch die Hydrierung hergestellte N,N-Dimethylaminopropylamin in einer weiteren Verfahrensstufe zur Herstellung von oberflächenaktiven Substanzen, Seifen, Kosmetik, Shampoos, Hygieneprodukten. Waschmittel und Pflanzenschutzmitteln eingesetzt wird.

## Claims

1. A process for hydrogenating nitriles by means of hydrogen in the presence of a catalyst in a reactor, where the catalyst is arranged in a fixed bed, wherein the cross-sectional loading in the reactor is in the range from 5 kg/ ($m^2$ s) to 50 kg/ ($m^2$ s) and 3-(dimethylamino)propionitrile or isophoronenitrilimine and/or isophoronenitrile are used as nitriles.

2. The process according to claim 1, wherein the fixed bed is a catalyst bed made up of loose, supported or unsupported shaped bodies.

3. The process according to at least one of claims 1 and 2, wherein the shaped bodies are used in the form of tablets, rings, cylinders, spheres or star extrudates.

4. The process according to at least one of claims 1 to 3, wherein the bulk density of the bed is from 0.1 to 3 kg/l.

5. The process according to at least one of claims 1 to 4, wherein the catalyst comprises Co or Ni.

6. The process according to at least one of claims 1 to 5, wherein the catalyst is produced by reduction of catalyst precursors.

7. The process according to at least one of claims 1 to 6, wherein part of the output (part output) from the hydrogenation reactor is recirculated as recycle stream to the reactor (circulating stream) and the ratio of circulating stream to feed stream fed in is in the range from 0.5:1 to 250:1.

8. The process according to at least one of claims 1 to 7, wherein the pressure is in the range from 15 to 85 bar and/or the temperature is in the range from 70 to 150°C.

9. The process according to at least one of claims 1 to 8, wherein the process is carried out in a shaft reactor, tube reactor or shell-and-tube reactor.

10. The process according to claim 9, wherein the ratio of height to diameter of the tube reactor is from 1:1 to 500:1.

11. The process according to at least one of claims 1 to 10, wherein the differential pressure over the catalyst bed is less than 1000 mbar/m.

12. The process according to at least one of claims 1 to 11, wherein isophoronenitrilimine or 3-(dimethylamino)propionitrile is used as nitrile.

**13.** The process according to at least one of claims 1 to 12, wherein isophoronenitrilimine is used as nitrile and the isophoronediamine prepared by hydrogenation is used in a further process stage for preparing hardeners for epoxy resins and coatings, specialty polyamides, polyurethanes and dyes.

**14.** The process according to at least one of claims 1 to 12, wherein 3-(dimethylamino)propionitrile is used as nitrile and the N,N-dimethylaminopropylamine prepared by hydrogenation is used in a further process stage for producing surface-active substances, soaps, cosmetics, shampoos, hygiene products, detergents and crop protection agents.

## Revendications

**1.** Procédé pour l'hydrogénation de nitriles par de l'hydrogène en présence d'un catalyseur dans un réacteur, le catalyseur étant disposé dans un lit fixe, **caractérisé en ce que** la charge sur la section transversale dans le réacteur se situe dans la plage de 5 kg/ ($m^2$.s) à 50 kg/ ($m^2$.s) et on utilise, comme nitrile, le 3-(diméthylamino)propionitrile ou l'isophoronenitrilimine et/ou l'isophorone-nitrile.

**2.** Procédé selon la revendication 1, **caractérisé en ce que** le lit fixe est une masse de catalyseur en vrac, constituée de corps façonnés en vrac, supportés ou non supportés.

**3.** Procédé selon au moins l'une quelconque des revendications 1 à 2, **caractérisé en ce que** les corps façonnés sont utilisés sous forme de comprimés, d'anneaux, de cylindres, de billes ou de brins en étoile.

**4.** Procédé selon au moins l'une quelconque des revendications 1 à 3, **caractérisé en ce que** la densité apparente de la masse en vrac est de 0,1 à 3 kg/l.

**5.** Procédé selon au moins l'une quelconque des revendications 1 à 4, **caractérisé en ce que** le catalyseur contient du Co ou du Ni.

**6.** Procédé selon au moins l'une quelconque des revendications 1 à 5, **caractérisé en ce que** le catalyseur est préparé par réduction de précurseurs de catalyseur.

**7.** Procédé selon au moins l'une quelconque des revendications 1 à 6, **caractérisé en ce qu'**une partie du produit sorti (sortie partielle) du réacteur d'hydrogénation est recyclée comme flux de recyclage dans le réacteur (flux en circulation) et le rapport du flux en circulation au flux alimenté de produits de départ se situe dans la plage de 0,5:1 à 250:1.

**8.** Procédé selon au moins l'une quelconque des revendications 1 à 7, **caractérisé en ce que** la pression est située dans la plage de 15 à 85 bars et/ou la température est située dans la plage de 70 à 150°C.

**9.** Procédé selon au moins l'une quelconque des revendications 1 à 8, **caractérisé en ce que** le procédé est réalisé dans un réacteur à cuve, un réacteur tubulaire ou un réacteur à faisceau tubulaire.

**10.** Procédé selon la revendication 9, **caractérisé en ce que** le rapport de la hauteur au diamètre du réacteur tubulaire et de 1:1 à 500:1.

**11.** Procédé selon au moins l'une quelconque des revendications 1 à 10, **caractérisé en ce que** la pression différentielle sur la masse de catalyseur en vrac est inférieure à 1000 mbars/m.

**12.** Procédé selon au moins l'une quelconque des revendications 1 à 11, **caractérisé en ce qu'**on utilise, comme nitrile, l'isophorone-nitrilimine ou le 3-(diméthylamino)propionitrile.

**13.** Procédé selon au moins l'une quelconque des revendications 1 à 12, **caractérisé en ce qu'**on utilise, comme nitrile, l'isophorone-nitrilimine et l'isophoronediamine préparée par l'hydrogénation est utilisée dans une autre étape de procédé pour la préparation de durcisseurs pour des résines époxyde et des revêtements, des polyamides particuliers, des polyuréthanes et des colorants.

**14.** Procédé selon au moins l'une quelconque des revendications 1 à 12, **caractérisé en ce qu'**on utilise, comme nitrile, le 3-(diméthylamino)propionitrile et la N,N-diméthylaminopropylamine préparée par hydrogénation est utilisée dans

une autre étape de procédé pour la préparation de substances tensioactives, de savons, de cosmétiques, de shampooings, de produits d'hygiène, d'agents de lavage et d'agents de phytoprotection.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 449089 A
- WO 2007128803 A
- EP 0636409 A
- EP 0742045 A
- EP 696572 A
- EP 963975 A
- EP 1106600 A2


### In der Beschreibung aufgeführte Nicht-Patentliteratur


- Amines, Aliphatic. Ullmann's Encyclopedia of Industrial Chemistry. 15. Juni 2000
- **A. B. STILES.** Catalyst Manufacture - Laboratory and Commercial Preperations. Marcel Dekker, 1983
- **ULLMANN.** Ullmann's Encyclopedia Electronic Release. 2000, 28-32
- **ERTL ; KNÖZINGER ; WEITKAMP.** Handbook of Heterogenoeous Catalysis. VCH Weinheim, 1997, 98
- **A. B. STILES.** Catalyst Manufacture. Marcel Dekker, Inc, 1983, 15
- **ERTL ; KNÖZINGER ; WEITKAMP.** Handbook of Heterogenoeous Catalysis. VCH Weinheim, 1997
- **ARTIKEL.** Fixed-Bed Reactors. *Ullmann's Encyclopedia of Industrial Chemistry,* 15. Juni 2000